# DEMANDE DE BREVET EUROPEEN

(11) **EP 3 875 124 A1**
(43) Date de publication de la demande: **08.09.2021**
(21) Numéro de dépôt: 20305213.9
(22) Date de dépôt: 02.03.2020
(51) Int. Cl.: A61L 27/22, A61L 27/38, A61L 27/60

(54) **PROCEDE D'OBTENTION D'UN TISSU DERMO-EPIDERMIQUE PRE-VASCULARISE**

(71) Demandeur: URGO RECHERCHE INNOVATION ET DEVELOPPEMENT, 21300 Chenôve (FR); Centre d'Etude des Cellules Souches (CECS), 91100 Corbeil Essonnes (FR); Universite d'Evry Val d'Essonne, 91000 Evry (FR)
(72) Inventeur: BALDESCHI, Christine, 91360 Villemoisson sur Orge (FR); DOMINGUES, Sophie, 91100 Corbeil-Essonnes (FR); LAURENSOU, Christelle, 21000 Dijon (FR); BOUSCHBACHER, Marielle, 21220 Chambolle Musigny (FR); CLEMENT, Sophie, 21160 Corcelles-les-Monts (FR); OLIVE, Guillaume, 21121 Fontaine-les-Dijon (FR)
(74) Mandataire: Plasseraud IP

(57) **Abrégé**

L'invention concerne un procédé d'obtention d'un substitut de peau comprenant les étapes suivantes :
a) mélange de fibroblastes, de cellules endothéliales et d'hydrogel d'origine exclusivement biologique ;
b) incubation du mélange obtenu à l'étape a) pendant une durée suffisante et dans conditions appropriées pour obtenir un derme pré-vascularisé ;
c) ajout de kératinocytes au derme pré-vascularisé de l'étape b) pour obtenir un substitut de peau ; dans lequel lesdits fibroblastes, cellules endothéliales et kératinocytes ont été obtenus à partir de cellules souches pluripotentes.

## Description

La présente invention concerne un procédé d'obtention d'un tissu dermo-épidermique pré-vascularisé à partir de kératinocytes, de fibroblastes et de cellules endothéliales dérivées de cellules souches embryonnaires ou de cellules souches induites à la pluripotence dans une matrice d'hydrogel d'origine exclusivement biologique.

### Contexte de l'invention

Les substituts cutanés sont des tissus artificiels créés en laboratoire, composés d'une matrice cellularisée ou non. Ces substituts cutanés sont capables de remplacer une partie de la peau absente ou défaillante chez des patients atteints de brûlures, de plaies chroniques, etc. ; ce qui constitue une alternative essentielle lorsque les traitements « standards » sont un échec. Le traitement standard actuel pour le traitement de ces plaies est la greffe de peau autologue, qui, tout en étant capable de couvrir le manque de tissu et restaurer la fonction de barrière en utilisant le tissu cutané du patient, présente l'inconvénient que le site de la plaie subit une contraction importante et un remodelage aléatoire des tissus.

La disponibilité d'une peau autologue est également une limitation dans les cas où les blessures d'un patient dépassent plus de 60% de sa surface corporelle totale ; dans ces cas, les blessures ne peuvent pas être couvertes de manière adéquate par les autogreffes en raison du manque de tissus prélevables. Le traitement nécessite donc l'utilisation de stratégies alternatives, le plus souvent des allogreffes cadavériques. Ceux-ci fonctionnent principalement comme un pansement temporaire pour protéger et stimuler la cicatrisation dans le lit de la plaie avant la pose d'une autogreffe.

Dans le cas des brûlures graves, infections nécrosantes des tissus mous, après un traumatisme, ou une résection tumorale, la quantité de tissu prélevé est importante. Dans la pratique clinique, de tels prélèvements sont généralement traités avec des greffes de peau autologues à double épaisseur. Cependant, en raison de la cicatrisation restrictive de ces greffons et de la capacité limitée de site donneur chez les patients gravement brûlés, il existe une forte demande pour des approches thérapeutiques alternatives. L'ingénierie des tissus cutanés est un domaine en croissance rapide en chirurgie plastique et reconstructive. Il se concentre sur l'implantation de substituts cutanés artificiels, qui devraient favoriser la cicatrisation rapide des plaies et prévenir la perte de liquide et les infections. De plus, les substituts cutanés devraient éviter les cicatrices et les contractures importantes.

Il existe sur le marché de nombreux substituts cutanés : Dermagraft ® (substitut dermique cellulaire à base de collagène, d'élastine et de fibroblastes allogéniques), MatriDerm® (substitut dermique acellulaire lyophilisé à base de collagène et élastine non réticulé), Apligraf® (substitut dermo-épidermique à base de collagène avec fibroblastes et kératinocytes primaires humains), etc. Cependant, tous ces pansements présentent plusieurs inconvénients, dont l'absence de cellules permettant la revascularisation rapide des zones touchées. En effet, la transition clinique de substituts cutanés a été limitée par la diffusion insuffisante d'oxygène et de nutriments dans le greffon. À des distances de diffusion supérieures à 150-200 µm, les constructions tissulaires sans réseau vasculaire perfusables subissent une mort cellulaire rapide et une nécrose. Par conséquent, afin d'obtenir une implantation réussie, le remplacement artificiel nécessite une perfusion et une intégration rapides avec le système vasculaire receveur.

De nombreuses équipes de recherche ont montré que l'ajout de cellules endothéliales dans les tissus générés *in vitro* (peau ou autres tissus/organes) permettait d'améliorer la prise de greffe et limiter la nécrose. La présence de ces cellules endothéliales permet la perfusion rapide du tissu greffé par anastomose avec les tissus de l'hôte.

La demande internationale WO2016/209166 décrit une méthode pour obtenir une couche de derme vascularisé en combinant les types cellulaires suivants, tous issus de cellules souches pluripotentes: des cellules endothéliales, des cellules musculaires lisses vasculaires/péricytes, et optionnellement des fibroblastes.

En particulier, la demande internationale WO2016/209166 enseigne qu'il est nécessaire d'associer des cellules musculaires lisses/péricytes aux cellules endothéliales afin d'obtenir une vascularisation fonctionnelle et pérenne.

De manière surprenante, les inventeurs ont démontré qu'il était possible d'obtenir un derme pré-vascularisé, et un tissu dermo-épidermique pré-vascularisé, à partir de cellules endothéliales et de fibroblastes issus de cellules souches pluripotentes, en l'absence de cellules musculaires lisses vasculaires/péricytes.

### Exposé de l'invention

La présente invention décrit un procédé de préparation de tissus dermo-épidermiques pré-vascularisés à partir de cellules dérivées de cellules souches pluripotentes, de préférence à partir de cellules dérivées de cellules souches pluripotentes humaines (hPSC ou « human pluripotent stem cells » en anglais) dans une matrice d'hydrogel d'origine exclusivement biologique.

Plus particulièrement, l'invention concerne un procédé d'obtention d'un substitut de peau comprenant les étapes suivantes :
a) mélange de fibroblastes, de cellules endothéliales et d'hydrogel d'origine exclusivement biologique;
b) incubation du mélange obtenu à l'étape a) pendant une durée suffisante et dans des conditions appropriées pour obtenir un tissu de type « derme pré-vascularisé » ;
c) ajout de kératinocytes au derme pré-vascularisé de l'étape b)- pour obtenir un substitut de peau
d) optionnellement, incubation à l'interface air-liquide pendant une durée suffisante pour obtenir un tissu dermo-épidermique pré-vascularisé pluristratifié
   dans lequel lesdits fibroblastes, cellules endothéliales et kératinocytes ont été obtenus à partir de cellules souches pluripotentes.

Selon un mode de réalisation particulièrement préféré, lesdits fibroblastes, cellules endothéliales et kératinocytes ont été obtenus à partir de cellules souches pluripotentes humaines.

Selon un mode de réalisation particulièrement préféré, aucune cellule musculaire lisse vasculaire (ou péricyte) n'est ajoutée au mélange de l'étape a).

Selon l'invention, l'incubation de l'étape b) a lieu pendant un temps suffisant et dans des conditions appropriées pour obtenir un derme pré-vascularisé.

Par « derme pré-vascularisé » ou « derme équivalent pré-vascularisé » ou « substitut de derme », on entend un tissu comprenant des fibroblastes et des cellules endothéliales inclus dans une matrice.

Par « tissu dermo-épidermique pré-vascularisé » «tissu dermo-épidermique » ou « substitut de peau », on entend un tissu comprenant un derme pré-vascularisé sur lequel repose une monocouche de kératinocytes.

Par « tissu dermo-épidermique pré-vascularisé pluristratifié », on entend un tissu comprenant un derme pré-vascularisé sur lequel repose un épiderme pluristratifié comprenant plusieurs couches de kératinocytes différenciés.

Par « hydrogel d'origine exclusivement biologique » on entend tout hydrogel comprenant des protéines d'origine animale ou végétale, tel que le chitosan, le collagène, l'élastine, la gélatine, la fibrine, les glycosaminoglycanes. Dans le cadre de la présente invention, l'hydrogel préféré est la fibrine plasmatique humaine.

Selon la présente invention, l'hydrogel ne contient pas de Poly Ethylene Glycol (PEG) ou d'autres polymères synthétiques fréquemment utilisés dans les matrices de substituts tissulaires.

Dans un mode de réalisation, l'étape b) a lieu pendant au moins 4 jours, de préférence au moins 6 jours, de manière encore plus préférée au moins 7 jours.

Dans un mode de réalisation, l'étape b) a lieu pendant au plus 14 jours, de préférence au au plus 12 jours, de manière encore plus préférée au plus 10 jours, 9 jours, 8 jours ou 7 jours.

Dans un mode de réalisation préféré de l'invention, l'étape b) a lieu pendant environ 7 jours.

Selon un mode de réalisation de l'invention, le milieu de culture de l'étape b) est composé de facteurs de croissance propices à la prolifération et à la maturation des fibroblastes et des cellules endothéliales. Par maturation, on comprend pour les fibroblastes : la production de matrice extracellulaire et le remodelage de la matrice ; et pour les cellules endothéliales, le remodelage de la matrice et l'assemblage d'un réseau vasculaire primitif.

Selon un mode de réalisation de l'invention, le milieu de culture de l'étape b) comprend du facteur de croissance endothélial vasculaire (« Vascular Endothelial Growth Factor » ou VEGF), de préférence du VEGF stabilisé. De manière préférée, le VEFG ou VEGF stabilisé, est présent dans le milieu de culture de l'étape b) à une concentration de 5 à 60 ng/mL, de préférence 45 à 55 ng/mL, de manière encore plus préférée environ 50 ng/mL.

Selon un mode de réalisation de l'invention, le milieu de culture de l'étape c), comprend des facteurs de croissance propices à la prolifération des kératinocytes et à leur éventuelle stratification ultérieure et à la stabilisation du réseau vasculaire primitif créé lors de l'étape b).

Selon un mode de réalisation de l'invention, le milieu de culture de l'étape c) comprend du VEGF, de préférence du VEGF stabilisé. De manière préférée, le VEFG ou VEGF stabilisé, est présent dans le milieu de culture de l'étape c) à une concentration de 5 à 60 ng/mL, de préférence 45 à 55 ng/mL, de manière encore plus préférée environ 50 ng/mL.

De manière avantageuse, les différents types cellulaires utilisés (fibroblastes, cellules endothéliales, kératinocytes) sont obtenus par différenciation de cellules souches pluripotentes, de préférence des cellules souches pluripotentes induites humaines.

Dans un mode de réalisation, l'invention ne repose pas sur l'utilisation de cellules nécessitant la destruction d'un embryon.

L'homme de l'art a, à sa disposition, plusieurs méthodes permettant d'obtenir des populations cellulaires différenciées homogènes à partir de cellules souches pluripotentes humaines.

De nombreux procédés de différentiation de cellules souches pluripotentes humaines en fibroblastes, cellules endothéliales ou kératinocytes ont été décrits dans la littérature.

Typiquement, des procédés de différentiation de cellules souches en fibroblastes sont décrits dans Shamis Y, Silva EA, Hewitt KJ, Brudno Y, Levenberg S, Mooney DJ, et al. (2013) Fibroblasts Derived from Human Pluripotent Stem Cells Activate Angiogenic Responses In Vitro and In Vivo. PLoS ONE 8(12): e83755.

Typiquement, des procédés de différentiation de cellules souches en cellules endothéliales ont été décrits dans les demandes EP3327118, WO2018101466 et US20180023051.

Typiquement, un procédé de différentiation de cellules IPS en kératinocytes est décrit dans la demande internationale WO2009/156398.

La présente invention couvre également les différentes utilisations, thérapeutiques et non thérapeutiques, desdits substituts de peau.

Dans un aspect, l'invention porte sur un substitut de peau tel que décrit ci-dessus en tant que médicament.

Dans un aspect, l'invention porte sur un substitut de peau tel que décrit ci-dessus pour une utilisation dans une méthode de traitement, tel que le traitement des brûlures et des plaies cutanées chroniques.

Dans un autre aspect, l'invention porte sur l'utilisation d'un substitut de peau tel que décrit ci-dessus dans une méthode de criblage de composés et molécules destinés à être en contact avec la peau et notamment à visée cosmétique ou thérapeutique.

Dans un autre aspect, la présente invention porte sur une population de substituts de peau obtenus selon le procédé décrit ci-dessus. De manière avantageuse, cette population de substituts de peau représente un ensemble d'échantillons homogène, reproductible, obtenu en conditions cliniques et en grande quantité.

### Brève description des Figures

D'autres caractéristiques, détails et avantages de l'invention apparaîtront à la lecture des Figures annexées.
**Fig. 1**

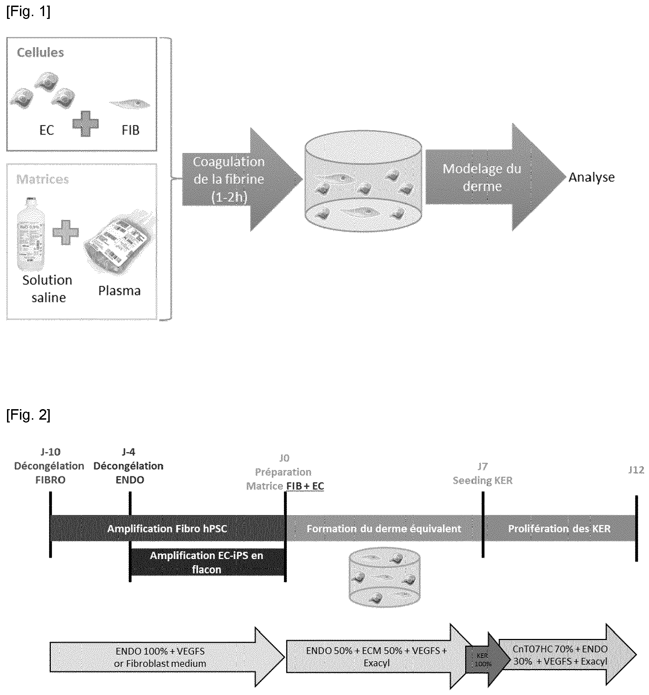

   [Fig. 1] représente le procédé d'obtention de derme équivalent à partir de cellules endothéliales, de fibroblastes et de fibrine.
**Fig. 2**
   [Fig. 2] représente un exemple de procédé d'obtention de tissu dermo-épidermique selon l'invention.
**Fig. 3**

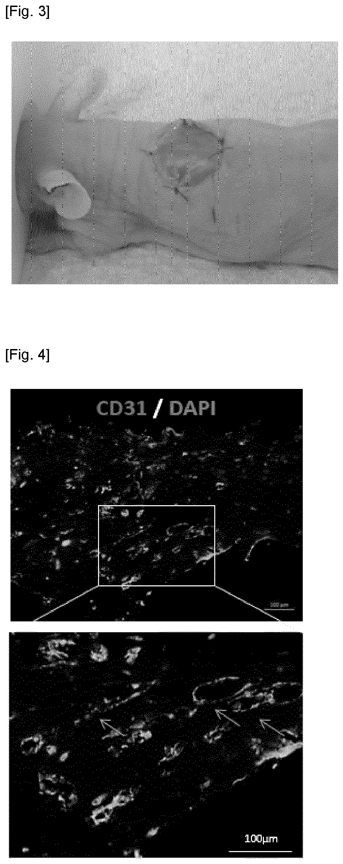

   [Fig. 3] représente un exemple de tissu dermo-épidermique selon l'invention suturée sur la Souris
**Fig. 4**
   [Fig. 4] représente la formation de structures circulaires composées de cellules endothéliales dans un tissu dermo-épidermique obtenu selon le procédé de l'invention dans lequel le biomatériau est de la fibrine et les fibroblastes et cellules endothéliales dérivés d'iPSC humaines sont ensemencés dans un ratio 1:40 (milieu EC :CNT)

### Exemples

### Exemple 1 : Obtention de fibroblastes, de cellules endothéliales et de kératinocytes à partir de cellules souches pluripotentes induites

Une lignée humaine de cellules souches induites à la pluripotence a été utilisée pour obtenir, par 3 procédés de différentiation adaptés, des fibroblastes (FIB), des cellules endothéliales (EC) et des kératinocytes (KER).

### Exemple 2 : Obtention d'un derme pre-vascularisé

Les fibroblastes (FIB) et les cellules endothéliales (EC) : les FIB et les EC ont été pré amplifiés en amont de la préparation des tissus en flacons de culture et en milieu F-CnT (CellnTec) pour les FIB et en milieu StemPro34 (Invitrogen) ou ENDO-CnT (CellnTec) supplémenté de VEGF à 50ng/mL pour les EC. Lorsque les 2 types cellulaires étaient prêts, ils ont été passés et comptés. Les cellules au ratio 1 FIB pour 40 EC ont été mélangées à la fibrine. La fibrine est obtenue en mélangeant du plasma et de la solution saline dans les conditions décrites dans le tableau 1 :

**[Tableau 1]**

| Composants de la fibrine | Concentrations initiales | Volume pour 5mL |
|---|---|---|
| Plasma humain | - | 2 mL |
| NaCl | 0,9% | 2,6 mL |
| Exacyl ® | 0,1g/mL | 20 µL |
| CaCl2 | 0,1 M | 400 µL |

La solution « cellules + fibrine » a été déposée dans des inserts de 1cm² soit 600µL/insert avec 10 000 FIB + 400 000 EC. Les plaques ont ensuite été mises à +37°C pour permettre la coagulation de la matrice pendant 1h minimum. Lorsque les fibrines ont pris, du milieu a été ajouté : 1mL dans les inserts et 2mL dans les puits afin que le tissu soit complètement immergé. Le milieu utilisé est un mélange de 1/3 de milieu F-CnT et 2/3 de milieu « EC » pendant 1 jour. Le lendemain (J1) et jusqu'à J7, le milieu a été changé tous les 2 jours par le milieu composé de 1/3 de milieu F-CnT et 2/3 de milieu EC, avec les mêmes volumes que cités.

### Exemple 3 : Obtention d'un tissu dermo-épidermique pre-vascularisé

La culture a été réalisée sur environ 14 jours à l'incubateur réglé à +37°C avec 5% CO₂ pour les tissus dermiques et 21 jours pour les tissus dermo-épidermiques. Le milieu F-CnT du mélange a été remplacé par le milieu ECM à J1 pour permettre aux FIB de sécréter de la matrice extracellulaire et du VEGF stabilisé à 50ng/mL.

A J7, une partie des tissus a été laissée dans les mêmes conditions jusqu'à J14 pour faire une analyse du derme seul et l'autre partie a été ensemencée avec des kératinocytes (KER) sur les matrices en milieu CnT07.HC avec 1.6mg/mL d'Exacyl® pendant 24-48h à 100 000/cm². A J9, les tissus avec KER ont été placés à l'interface air-liquide pour permettre la stratification de l'épiderme. Le milieu utilisé était alors composé de milieu Airlift à 70%, de milieu ENDO-CnT à 30% avec de l'Exacyl® à 1.6mg/mL et du VEGF stabilisé à 50ng/mL.

Ces expériences ont permis d'obtenir un tissu dermo-épidermique pré-vascularisé qui, une fois greffée sur la Souris, ne présentait pas de nécrose.

## Revendications

1. Procédé d'obtention d'un substitut de peau comprenant les étapes suivantes :
a) mélange de fibroblastes, de cellules endothéliales et d'hydrogel d'origine exclusivement biologique;
b) incubation du mélange obtenu à l'étape a) pendant une durée suffisante et dans des conditions appropriées pour obtenir un tissu de type « derme pré-vascularisé » ;
c) ajout de kératinocytes au derme pré-vascularisé de l'étape b)- pour obtenir un substitut de peau
d) optionnellement, incubation à l'interface air-liquide pendant une durée suffisante pour obtenir un tissu dermo-épidermique pré-vascularisé pluristratifié
dans lequel lesdits fibroblastes, cellules endothéliales et kératinocytes ont été obtenus à partir de cellules souches pluripotentes.

2. Procédé selon la revendication 1 **caractérisé en ce qu'**aucune cellule musculaire lisse vasculaire n'est ajoutée au mélange de l'étape a).

3. Procédé selon la revendication 1 ou 2 **caractérisé en ce que** l'incubation de l'étape a) a lieu pendant 7 jours.

4. Procédé selon l'une quelconque des revendications 1 à 3, **caractérisé en ce que** du facteur de croissance épidermique vasculaire (VEGF), de préférence du VEGF stabilisé, est présent dans le milieu de culture de l'étape b), de préférence à une concentration de 30 à 60 ng/mL, de préférence 45 à 55 ng/mL.

5. Procédé selon l'une quelconque des revendications 1 à 4, **caractérisé en ce que** du VEGF, de préférence du VEGF stabilisé, est présent dans le milieu de culture de l'étape c), de préférence à une concentration de 30 à 60 ng/mL, de préférence 45 à 55 ng/mL.

6. Procédé selon l'une quelconque des revendications précédentes, **caractérisé en ce que** les fibroblastes et les cellules endothéliales de l'étape a) sont obtenus à partir de cellules souches pluripotentes induites humaines.

7. Procédé selon l'une quelconque des revendications précédentes, **caractérisé en ce que** les kératinocytes de l'étape c) sont obtenus à partir de cellules souches pluripotentes induites humaines.

8. Substitut de peau comprenant un derme pré-vascularisé et une monocouche de kératinocytes obtenu selon le procédé de l'une quelconque des revendications 1 à 7.

9. Substitut de peau comprenant un derme pré-vascularisé et un épiderme pluristratifié, obtenu selon le procédé de l'une quelconque des revendications 1 à 7.

10. Substitut de peau selon la revendication 8 ou 9 pour son utilisation en tant que médicament.

11. Substitut de peau selon la revendication 8 ou 9 pour son utilisation dans une méthode de traitement.

12. Utilisation d'un substitut de peau selon la revendication 8 ou 9 dans une méthode de criblage de composés et molécules destinés à être en contact avec la peau et notamment à visée cosmétique et/ou thérapeutique.

13. Population de substituts de peau obtenus par le procédé selon l'une quelconque des revendications 1 à 7.
